# EUROPEAN PATENT APPLICATION

(11) **EP 0 800 806 A2**
(43) Date of publication of application: **15.10.1997**
(21) Application number: 97104179.3
(22) Date of filing: 12.03.1997
(51) Int. Cl.: A61F 5/48, A61F 13/36

(54) **A fabric for medical use, in particular for use as a stretcher or sling**

(30) Priority: 12.03.1996 GB 9605230
(71) Applicant: ROTECNO AG, CH-8045 Zürich (CH)
(72) Inventor: Patel, Suresh R., Dr., Dalgety Bay, Scotland KY11 5YL (GB); Duncan, John, Dr., Glenrothes, Scotland KY6 2QB (GB)
(74) Representative: Morgan, James Garnet

(57) **Abstract**

A fabric for medical use comprises an upper layer and a lower layer and preferably also an intermediate layer. The upper layer is woven or knitted and adapted to minimise damage to a patient's skin when disposed against the skin of the patient. The lower layer is resistant to the passage of liquid, more specifically to the passage of body fluids, to prevent such body fluids passing through a lower surface of the lower layer. The fabric is typically used above a base layer fabric to form a stretcher or patient transfer sling.

## Description

This invention relates to a novel fabric for use for medical purposes such as for a patient transfer sling, a stretcher, or a blanket to be disposed next to a patient's skin.

Stretchers are typically made from cotton canvas having poles disposed on the sides to allow the carrying of a patient on the stretcher. More elaborate systems have mechanical carrying methods, such as a trolley with wheels but still utilise the basic design of a strong canvas type fabric hung between two poles. The contamination of stretchers etc., e.g. by blood or body fluids from injured patients is a problem, which has hitherto not been adequately addressed. Incidences of skin damage and infection have also been reported in cases where patients remain on conventional stretchers for long periods. There has been great consideration for special mattresses and beds (see, for example, J. Fletcher (1996), Types of Pressure-Relieving Equipment Available, British Journal of Nursing, Vol. 5, No. 13, 810-6).

Similar problems arise with so-called slings, which are frequently used to facilitate the transfer of patients from hospital beds to operating theatres and back to the hospital bed. This type of patient handling is in particular frequently used with immobile patients.

During an operation, which can last for many hours, the patient is generally given an anaesthetic, which frequently includes a muscle relaxant, and which results in suppressed circulation of the patient's blood. The combination of this and a protracted period of contact with a sling can cause substantial problems with respect to bed sores and irritation of the patient's skin.

Such bed sores or damage to the patient's skin can also lead to infection of a serious nature. Such bed sores or pressure sores also tend to occur more frequently in the elderly, as can be seen, for example, by the article of M. Versluysen (1986), How Elderly Patients with Femoral Fractures Develop Pressure Sores, in the British Medical Journal 292, 1311-13.

It is a principle object of the present invention to provide a fabric which minimises the danger of skin damage, which is able to cope with body fluid loss, which counteracts the creation of pressure sores and which also facilitates nurses in transferring immobile patients to and fro beds and avoids the incidence of back injuries in such nursing staff.

In order to satisfy the above object, there is provided, in accordance with the present invention, a fabric for medical use, comprising an upper layer and a lower layer, said upper layer being woven or knitted and adapted to minimise damage to a patient's skin when disposed against the skin of a patient, and said lower layer being resistant to the passage of liquid, most specifically the passage of body fluids, whereby to prevent such body fluids passing through a lower surface of said lower layer.

With a fabric of this kind it is possible to select the properties of the upper layer so that the patient's skin is in contact with a soft material, which treats the patient's skin gently and which permits body fluids lost from the patient to pass through to the lower layer. The lower layer, which can be absorbent at its upper surface and sealed at its lower surface, is able to absorb the body fluid and simultaneously to prevent it from being transferred to lower lying fabrics, such as bed sheets or stretchers or slings.

A particularly preferred embodiment involves three layers of fabric and has, in particular, an intermediate layer which is chosen for its liquid absorbent properties.

The fabric, irrespective of whether it is a two-layer fabric or a three-layer fabric, can then be used above a base layer fabric to form a stretcher or patient transfer sling. The fabric may be permanently or releasably joined to the base layer fabric to form the stretcher or sling.

In a particularly preferred fabric the upper layer is of synthetic material, preferably polyester. It may, for example, comprise a loop-raised, warp-knit polyester, in particular a loop-raised, warp-knit polyester of 50 dtex, 20 filament yarns having 51 cpi courses (courses per inch) and 40 wpi (wales per inch), said filaments preferably being endless filaments. Alternatively, it may be a woven loop-pile fabric, such as a terry fabric. The intermediate absorbent layer is preferably an absorbent fabric material of synthetic endless fibres as described and claimed in European patent 710 303.

The lower layer is generally a waterproof material and preferably comprises a coated, warp-knit fabric, again preferably of polyester, said fabric having a coating of polyurethane of a type resistant to hydrolysis to withstand repeated washing. The polyurethane coating seals the warp-knit fabric, at least at the lower side thereof, to produce a membrane largely impervious to body fluids.

A structure of this kind has the advantage that it can be laundered in accordance with Hospital Laundry Regulations (it does not have to be sterilised), many times without differential shrinkage occurring, leading to premature failure of the fabric.

In use, the fabric is typically used with a base layer fabric comprising a woven nylon fabric which has in particular high strength for coping with the mechanical loads applied by patients when being manoeuvred by nursing staff on the sling or stretcher.

It is also conceivable for the base layer fabric to form the lower layer of a three layer fabric.

Further particularly preferred embodiments of the invention are set forth in the subordinate claims.

Thus, according to the present invention, there is provided a fabric for medical purposes, the fabric having an upper surface which is woven or knitted so as to minimise damage to a patient's skin when disposed against the skin of said patient, and a lower layer which is resistant to the passage of liquid.

Preferably, the fabric has an intermediate layer between the upper surface and the lower layer, the intermediate layer being liquid absorbent.

The preferred embodiment of the invention comprises a stretcher or patient transfer sling which comprises the fabric. The fabric may overlie the top of the stretcher or sling, or may form an integral part thereof. In the latter case, the fabric may be combined with a base layer having high strength and preferably being flat. Such a base layer may comprise synthetic material such as woven nylon etc., and may incorporate handles for manipulation of the sling or stretcher, or channels for receiving poles.

Thus, a preferred embodiment of the invention provides a fabric for medical purposes, the fabric having an upper surface which is woven or knitted so as to minimise damage to a patient's skin when disposed against the skin of said patient, a lower layer which is resistant to the passage of liquid, and a base layer.

At least the upper surface of the fabric is preferably of synthetic material such as polyester, and can advantageously comprise knitted raised loop polyester fabric. The construction of the upper surface from appropriate synthetic fabrics can help to minimise fluid absorption by the upper surface and provide good soil release properties when the fabric is laundered.

Where the fabric comprises an intermediate layer, it is especially advantageous for the upper surface to be non-absorbent and to transfer liquid efficiently to the absorbent intermediate layer.

The upper surface, and preferably the whole fabric, is advantageously non-linting which minimises open wound contamination and provides an advantage over the use of natural fabrics such as cotton canvas which are subject to linting.

When present, the absorbent intermediate layer is preferably bonded to the liquid resistant lower layer, such as by coating the intermediate layer with a liquid resistant coating. The intermediate layer can itself comprise several layers of absorbent fabric. Where the lower layer and the intermediate layer(s) are of a multi-layered construction.

They are preferably attached (e.g. by bonding) together at the edges.

The lower layer preferably has a water penetration resistance greater than 150cm hydrostatic head of water.

The fabric is preferably capable of being laundered at 71°C as specified in Health Service Guidelines, Hospital Laundry Arrangements for Used and Infected Linen, Document No HSG95/18.

Where the fabric is of multi-layer construction, the layers are preferably attached at their edges so that the water resistant properties of the lower layer are not compromised by stitching or other means of joining the layers. The fabric may be bonded together by adhesive or such other means which obviates the requirement for sewing.

A sling and/or a stretcher comprising the fabric may be provided with handles to allow the manipulation of patients without removing the patients from the sling/stretcher, and to minimise back injury for nurses etc. The handles are preferably attached along the outside edge, and are preferably disposed against the lower surface of the lower layer such that the fabric is disposed between the patient and the handles, thereby minimising patient trauma. Alternatively and/or additionally, the sling/stretcher may have flaps extending for some distance form the sides thereof, which can wrap around the patient and can be fixed together e.g. by fasteners.

A stretcher of the invention may have two channels in the bottom layer of the fabric to receive poles for carrying the patient. The stretcher/sling is useful for the transfer of a patient from the location of an accident to a treatment centre, and between various locations in the hospital. The patient can be transported and treated on the stretcher/sling and can remain on the same stretcher/sling throughout a surgical procedure in an operating theatre. Where the patient is transported from the site of an accident using a stretcher of the invention, the poles can simply be removed when the patient is transferred onto an operating theatre table. The stretcher of the invention may be used in conjunction with a stretcher carrier which supports the poles on wheel-mounted bases.

This has the benefit that the patient, who may have open wounds etc., is in contact only with the upper surface of the fabric on the stretcher and the skin damage to the patient and the transfer of particulates to the wounds of the patient is minimised. Any blood etc. emanating from the patient may also be contained in/on the fabric.

Embodiments of the present invention will now be described by way of example only and with reference to the accompanying drawings in which:
- Fig. 1: shows a plan view of a stretcher according to one embodiment of the invention;
- Fig. 2: shows a sectional view through the stretcher of Fig. 1;
- Fig. 2a: shows an alternative design of fabric;
- Fig. 3a: shows a plan view of a second stretcher;
- Fig. 3b: shows an end view of the Fig. 3a stretcher;
- Fig. 4a: shows an end view of the Fig. 3 stretcher in use; and
- Fig. 4b: shows a plan view of the Fig. 3 stretcher in use.

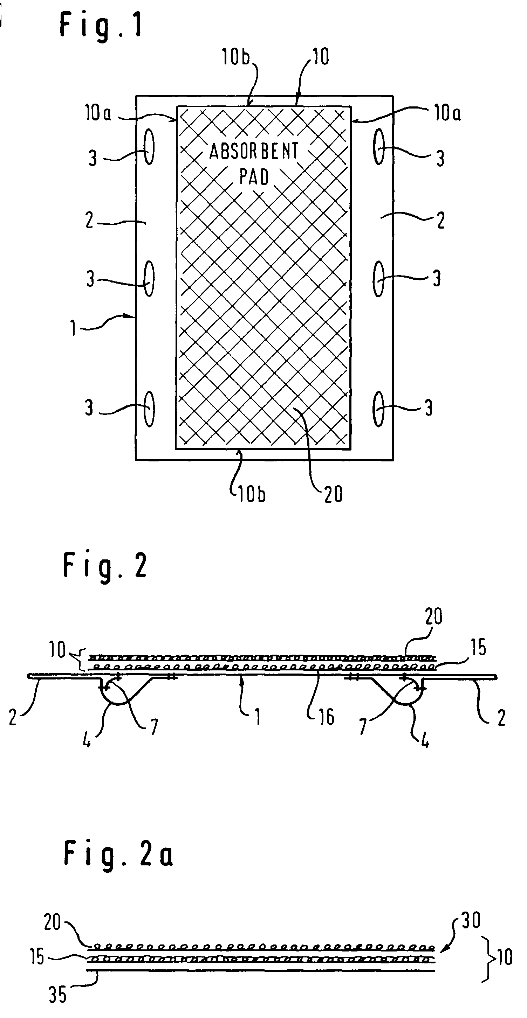

Referring now to the drawings, Fig. 1 shows a stretcher having a base layer fabric 1 (optionally of liquid impermeable fabric) being sufficiently strong to support the weight of a patient. Advantageously the base layer 1 may be of woven nylon and may be folded back at the longitudinal sides as shown in Fig. 2. The base layer 1 has side flaps 2 into which are sewn or cut handles 3. The underside of the base layer 1 has channels 4 incorporated therein for receiving stretcher poles (not shown). The channels are reinforced at 7, e.g. by the same woven nylon as is used for the base layer 1.

On the upper surface of the base layer 1 there is an absorbent pad 10 comprising an upper layer 20 which forms the upper surface of the stretcher and an intermediate layer 15 disposed between the upper layer and the base layer fabric 1. The absorbent pad 10 is of a sufficient length and width to underlie the entire body of a patient placed on the stretcher. The size of the base layer 1 is preferably chosen so as to underlie all of the absorbent pad 10, and preferably to extend beyond the absorbent pad 10 for a short distance at each edge 10a, 10b.

The upper layer 20 is a synthetic polyester knitted fabric with a raised surface which is non-linting and is soft and non-damaging to the patient's skin. The upper layer 20 has little if any liquid absorptive properties and allows liquid such as blood etc. emanating from the patient to pass through to the intermediate layer 15. More specifically, the top or upper layer, which has the function of permitting the passage of liquids and of being comfortable against the patients' skin, preferably comprises a loop-raised warp-knit polyester of 50 dtex with 20 filament yarns, having 51 cpi per 25 mm and 40 wpi per 25 mm.

The intermediate layer 15 may itself have a liquid-resistant coating on the underside thereof so as to prevent transfer of liquids etc. through to the base layer 1. In this case, the liquid-resistant coating can be thought of as a lower layer. Indeed, a conventional stretcher comprising a cotton canvas stretcher cover and poles may simply be overlaid by a pad of the fabric (optionally with an absorbent intermediate layer) with a liquid-resistant layer on the lower surface thereof. In such cases the lower surface of the absorbent pad may optionally be reinforced so as to allow transfer of the patient on the absorbent pad, and may incorporate handles etc.

The absorptive second layer 15 is also preferably of woven synthetic fabric and may be chemically treated to increase its absorptive properties. Such treatments are known in the art. The intermediate absorbent second layer 15 is preferably an absorbent fabric described and claimed in the applicants' European patent 710 303. A preferred second layer 15 comprises high temperature wash-resistant polyurethane coated to protect the absorbent fabric.

Fig. 2a shows a preferred embodiment of the present invention, comprising a three-layer fabric 30, comprising an upper layer 20 and an intermediate layer 15 as previously described, with a bottom layer, which is a waterproof coated warp-knit fabric. Here the fabric of the bottom layer, which may also comprise polyester, is coated with polyurethane of a type resistant to hydrolysis to withstand repeated washing. The fabric of Fig. 2a forms an absorbent pad 10 which may be mounted onto a base layer fabric, such as fabric 2 shown in Fig. 2, to form a stretcher or sling. In this case the base fabric 1 is, for example, a high-tenacity woven nylon plain weave fabric of 1055 dtex yarn. The stretcher fabric has a break strength of 4067 N at 47 % elongation in the warp direction (longitudinal direction) and a break strength of 3030 N, again at 47 % elongation, in the warp direction. In use the strong fabric for the base would typically be oriented with the warps in the longitudinal direction of the stretcher. The arrangement could, however, be such that the weft threads are aligned with the longitudinal direction of the stretcher.

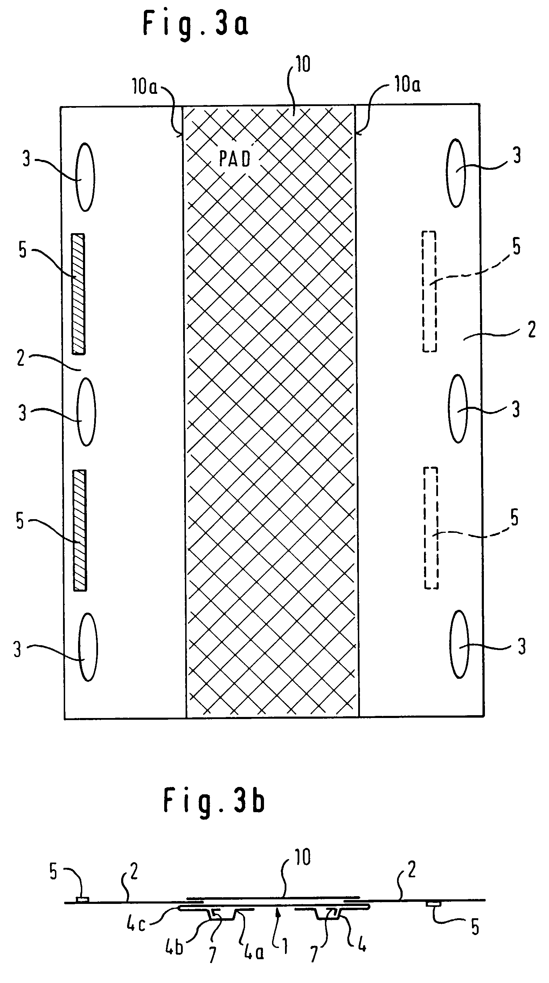

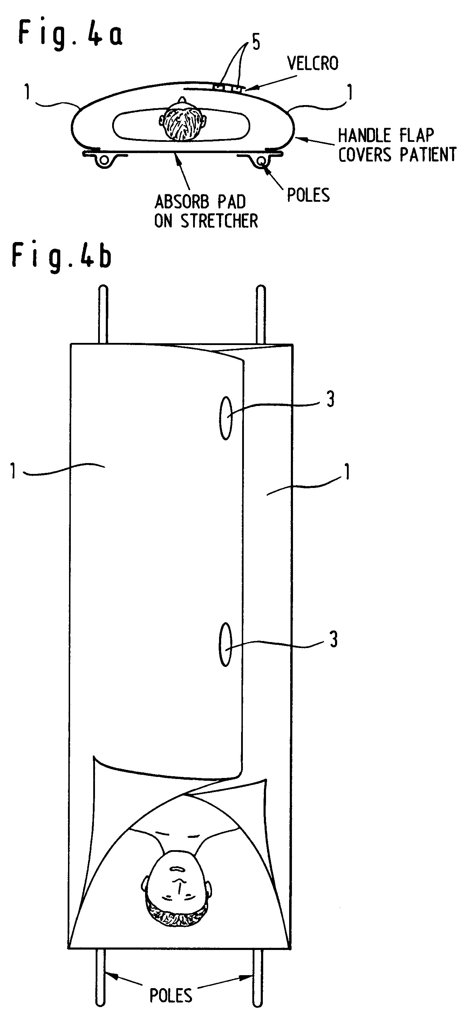

Figs. 3 and 4 show a stretcher or sling in which the flaps 2 extend for some 50cm from each side 10a of the pad 10, and have fixing means such as Velcro (TM) tabs 5 at the edges so that the flaps 2 can be wrapped around a patient on the stretcher and fastened together. The base layer 1 and flaps 2 are fabricated from separate pieces. The base layer 1 is composed of a single piece of woven nylon which is folded into loops 4 and sewn or otherwise bonded to itself at 4a, 4b and 4c. The joint at 4b is reinforced (7) with a strip of woven nylon so that the stretcher is strongest at the regions of contact with the poles when used as a stretcher .

If there is no desire to use the structure of Figures 3 and 4 as a stretcher, then the channels for the poles can be omitted, although it is useful, but not essential, to double back the edges of the base layer fabric for added strength.

The pad 10, which may be the pad 10 of Fig. 2 or of Fig. 2a, can be attached to the stretcher of the second embodiment only at the edges 10a, 10b, and this has the advantage that the water-resistance of the pad 10 is not compromised.

Certain embodiments of the present invention can help to contain body fluids emanating from a patient and minimise the contamination of personnel and medical equipment such as ambulances, operating tables etc.

The handles on the outer flaps 2 of the stretcher or fabric are attached thereto in a manner which avoids forming ridges underneath the absorbent pad which could give rise to sites of local pressure and abrasion and also avoids compromising the liquid resistant properties of the absorbent pad 10 when provided with a liquid resistant base layer.

In certain embodiments of the invention the absorbent pad covers the channels for the poles.

If the fabric of the invention is used for a sling only, then the lower layer of the three-layer fabric can also form the base layer which provides the strength required for manipulation of the patient. The structure then closely resembles Fig. 2, but the channels for the poles 4 could be omitted. I.e., the structure of Fig. 2 is used as a three-layer fabric to form a sling. In this case the base layer 1 can itself comprise the coated warp-knit fabric coated with polyurethane.

Modifications and improvements may be incorporated without departing from the scope of the invention.

## Claims

1. Fabric for medical use, comprising an upper layer and a lower layer, said upper layer being woven or knitted and adapted to minimise damage to a patient's skin when disposed against the skin of a patient, and said lower layer being resistant to the passage of liquid, most specifically the passage of body fluids, whereby to prevent such body fluids passing through a lower surface of said lower layer.

2. Fabric in accordance with claim 1, comprising an intermediate layer disposed between said upper layer, or at least said upper surface and said lower layer, said intermediate layer being liquid-absorbent.

3. Fabric in accordance with claim 1 or claim 2, when used above a base layer fabric to form a stretcher or patient transfer sling.

4. Fabric in accordance with claim 3, wherein the first said fabric is permanently or releasably joined to said base layer fabric to form said stretcher or sling.

5. Fabric in accordance with any one of the preceding claims, wherein said base layer fabric is of a strength adequate to reliably support a patient, and optionally forms the lower layer of a three layer fabric.

6. Fabric in accordance with any one of the preceding claims, wherein said base layer fabric is provided with or formed into handles for handling of the sling with a patient thereon and/or is provided with or formed into channels for receiving poles for use as a stretcher.

7. Fabric in accordance with any one of the preceding claims, wherein said upper layer is of synthetic material, preferably polyester.

8. Fabric in accordance with claim 7, wherein said upper layer comprises a loop-raised, warp-knit polyester, in particular a loop-raised, warp-knit polyester of 50 dtex, 20 filament yarns having 51 cpi courses (courses per inch) and 40 wpi wales (wales per inch), said filaments preferably being endless filaments.

9. Fabric in accordance with claim 7, wherein said upper layer comprises a woven loop-pile fabric, such as a terry fabric.

10. Fabric in accordance with any one of the preceding claims, wherein said intermediate, absorbent layer comprises a fabric in accordance with any one of the claims of European patent 710 303 as granted, and more specifically comprises an absorbent fabric material of synthetic endless fibres, wherein the fibre of the fabric material can be bulked and the woven material has an open woven structure in which longer non-bound thread sections alternate with firmly bound-in thread sections, and wherein preferably a highly bulkable fibre such as false twist extruded or friction extruded polyester yarn is used as the synthetic endless fibre, e.g. having individual filaments with a thickness from 0.5 dtex to 5 dtex, in particular of 1 dtex to 2.5 dtex, i.e. grams per 10,000 m, and with the synthetic endless fibres having a total thickness of 50 dtex to 500 dtex..

11. Fabric in accordance with any one of the preceding claims, wherein said lower layer comprises a waterproof material and preferably comprises a coated, warp-knit fabric, said fabric having a coating of polyurethane of a type resistant to hydrolysis to withstand repeated washing.

12. Fabric in accordance with any one of the preceding claims, wherein said base layer fabric comprises a woven nylon fabric, preferably a high tenacity nylon woven plain weave of 1055 dtex yarn with the fabric of the base layer having a warp strength such that break occurs at approximately 4067 N at 47 % elongation and a weft strength such that break occurs at approximately 3030 N at 47 % elongation.

13. Fabric in accordance with claim 10, wherein the layers of the first said fabric are stitched or bonded together at their edges, and are also stitched, bonded together or releasably secured to said base layer fabric at their edges.

14. Fabric in accordance with any one of the preceding claims, wherein said intermediate layer and/or said lower layer comprise a multi-layered construction.

15. Fabric in accordance with any one of the preceding claims, wherein said lower layer has a water-penetration resistance greater than 150 cm of hydrostatic head of water.

16. Fabric in accordance with any one of the preceding claims, capable of being laundered at 71°C as specified in British Health Service Guidelines, Hospital Laundry Arrangements for Used and Infected Linen, document No. HSG 95/18.

17. Fabric in accordance with any one of the preceding claims, wherein said handles are disposed against the lower surface of the base layer fabric so that the fabric is disposed between the patient and the handles.

18. Fabric in accordance with any one of the preceding claims, when used as a sling or stretcher and having flaps extending from its longitudinal sides and adapted to be wrapped around a patient, there being preferably connection means such as fasteners, in particular loop and hook fasteners (Velcro TM) or ties on said flaps for connecting the flaps together, e.g. around a patient or for storage.

19. Fabric in accordance with any one of the preceding claims, wherein the upper layer is connected (optionally releasably connected) to the lower layer and optionally to an intermediate layer provided between the upper layer and lower layer, and these two or three layers are releasably connected to the base fabric layer.

20. Fabric when used for a stretcher, wherein said base layer fabric is folded back on itself at its longitudinal edges and stitched to itself to form open pockets for receiving poles, and wherein a reinforcement member, optionally of the same material as said base layer fabric, is stitched into the turned back portion of said base layer fabric for reinforcement, and wherein, in the event of the provision of flaps, these protrude beyond the longitudinal sides of the base layer fabric, and comprise either the fabric mounted on said base layer fabric or a different material.

21. Fabric in accordance with any one of the preceding claims, wherein said absorbent layer is a disposable layer.
